# FASCICULE DE BREVET EUROPEEN

(11) **EP 1 124 577 B1**
(45) Date de publication et mention de la délivrance du brevet: **27.08.2003**
(21) Numéro de dépôt: 99971719.2
(22) Date de dépôt: 08.11.1999
(51) Int. Cl.: A61K 39/385, A61K 39/39, A61P 31/00, A61P 35/00, A61P 37/00

(54) **UTILISATION D'UNE PROTEINE OMPA D'ENTEROBACTERIE, POUR LE CIBLAGE SPECIFIQUE VERS LES CELLULES PRESENTATRICES D'ANTIGENES**
Verwendung von OmpA Enterobakterproteinen um zielgerichtet Antigen-Präsentierende Zellen anzusteuern
USE OF AN ENTEROBACTERIUM PROTEIN OmpA FOR SPECIFIC TARGETING TOWARDS ANTIGEN-PRESENTING CELLS

(30) Priorité: 06.11.1998 FR 9814007
(43) Date de publication de la demande: 22.08.2001
(73) Titulaire: PIERRE FABRE MEDICAMENT, 92100 Boulogne-Billancourt (FR)
(72) Inventeur: BONNEFOY, Jean-Yves, F-74350 Le Sappey (FR); LECOANET, Sybille, CH-1196 Gland (CH); AUBRY, Jean-Pierre, F-74350 Cuvat (FR); JEANNIN, Pascale, F-01220 Divonne-les-Bains (FR); BAUSSANT, Thierry, F-01200 Bellegarde (FR)
(74) Mandataire: Ahner, Francis
(86) Numéro de dépôt international: FR9902734
(87) Numéro de publication internationale: WO00027432

(56) Documents cités:
- WO-A-96/14415
- WO-A-97/41210
- WO-A-97/41888
- HAEUW J F ET AL: "The recombinant Klebsiella pneumoniae outer membrane protein OmpA has carrier properties for conjugated antigenic peptides." EUROPEAN JOURNAL OF BIOCHEMISTRY, (1998 JUL 15) 255 (2) 446-54., XP002114947

### LISTE DE SÉQUENCES

<110> PIERRE FABRE MÉDICAMENT
<120> UTILISATION D'UNE PROTÉINE OmpA D'ENTÉROBACTÉRIE, POUR LE CIBLAGE SPÉCIFIQUE D'UNE SUBSTANCE BIOLOGIQUEMENT ACTIVE QUI LUI EST ASSOCIÉE VERS LES CELLULES PRÉSENTATRICES D'ANTIGÈNES

<130> D17777

<140>
<141>

<150> FR 98 14007
<151> 1998-11-06

<160> 2

<170> PatentIn Ver. 2.2

<210> 1
<211> 1035
<212> ADN
<213> Klebsiella pneumoniae

<220>
<221> exon
<222> (1)..(1032)

<220>
<221> intron
<222> (1033)..(1035)

<220>
<221> CDS
<222> (1)..(1032)
<400> 1
   atg aaa gca att ttc gta ctg aat gcg gct ccg aaa gat aac acc tgg     48
   Met Lys Ala Ile Phe Val Leu Asn Ala Ala Pro Lys Asp Asn Thr Trp
     1               5                  10                  15
   tat gca ggt ggt aaa ctg ggt tgg tcc cag tat cac gac acc ggt ttc     96
   Tyr Ala Gly Gly Lys Leu Gly Trp Ser Gln Tyr His Asp Thr Gly Phe
                20                  25                  30
   tac ggt aac ggt ttc cag aac aac aac ggt ccg acc cgt aac gat cag    144
   Tyr Gly Asn Gly Phe Gln Asn Asn Asn Gly Pro Thr Arg Asn Asp Gln
            35                  40                  45
   ctt ggt gct ggt gcg ttc ggt ggt tac cag gtt aac ccg tac ctc ggt    192
   Leu Gly Ala Gly Ala Phe Gly Gly Tyr Gln Val Asn Pro Tyr Leu Gly
        50                  55                  60
   ttc gaa atg ggt tat gac tgg ctg ggc cgt atg gca tat aaa ggc agc    240
   Phe Glu Met Gly Tyr Asp Trp Leu Gly Arg Met Ala Tyr Lys Gly Ser
    65                  70                  75                  80
   gtt gac aac ggt gct ttc aaa gct cag ggc gtt cag ctg acc gct aaa    288
   Val Asp Asn Gly Ala Phe Lys Ala Gln Gly Val Gln Leu Thr Ala Lys
                       85                  90                  95
   ctg ggt tac ccg atc act gac gat ctg gac atc tac acc cgt ctg ggc    336
   Leu Gly Tyr Pro Ile Thr Asp Asp Leu Asp Ile Tyr Thr Arg Leu Gly
               100                 105                 110
   ggc atg gtt tgg cgc gct gac tcc aaa ggc aac tac gct tct acc ggc    384
   Gly Met Val Trp Arg Ala Asp Ser Lys Gly Asn Tyr Ala Ser Thr Gly
           115                 120                 125
   gtt tcc cgt agc gaa cac gac act ggc gtt tcc cca gta ttt gct ggc    432
   Val Ser Arg Ser Glu His Asp Thr Gly Val Ser Pro Val Phe Ala Gly
       130                 135                 140
   ggc gta gag tgg gct gtt act cgt gac atc gct acc cgt ctg gaa tac    480
   Gly Val Glu Trp Ala Val Thr Arg Asp Ile Ala Thr Arg Leu Glu Tyr
   145                 150                 155                 160
   cag tgg gtt aac aac atc ggc gac gcg ggc act gtg ggt acc cgt cct    528
   Gln Trp Val Asn Asn Ile Gly Asp Ala Gly Thr Val Gly Thr Arg Pro
                   165                 170                 175
   gat aac ggc atg ctg agc ctg ggc gtt tcc tac cgc ttc ggt cag gaa    576
   Asp Asn Gly Met Leu Ser Leu Gly Val Ser Tyr Arg Phe Gly Gln Glu
               180                 185                 190
   gat gct gca ccg gtt gtt gct ccg gct ccg gct ccg gct ccg gaa gtg    624
   Asp Ala Ala Pro Val Val Ala Pro Ala Pro Ala Pro Ala Pro Glu Val
           195                 200                 205
   gct acc aag cac ttc acc ctg aag tct gac gtt ctg ttc aac ttc aac    672
   Ala Thr Lys His Phe Thr Leu Lys Ser Asp Val Leu Phe Asn Phe Asn
       210                 215                 220
   aaa gct acc ctg aaa ccg gaa ggt cag cag gct ctg gat cag ctg tac    720
   Lys Ala Thr Leu Lys Pro Glu Gly Gln Gln Ala Leu Asp Gln Leu Tyr
   225                 230                 235                 240
   act cag ctg agc aac atg gat ccg aaa gac ggt tcc gct gtt gtt ctg    768
   Thr Gln Leu Ser Asn Met Asp Pro Lys Asp Gly Ser Ala Val Val Leu
                   245                 250                 255
   ggc tac acc gac cgc atc ggt tcc gaa gct tac aac cag cag ctg tct    816
   Gly Tyr Thr Asp Arg Ile Gly Ser Glu Ala Tyr Asn Gln Gln Leu Ser
               260                 265                 270
   gag aaa cgt gct cag tcc gtt gtt gac tac ctg gtt gct aaa ggc atc    864
   Glu Lys Arg Ala Gln Ser Val Val Asp Tyr Leu Val Ala Lys Gly Ile
           275                 280                 285
   ccg gct ggc aaa atc tcc gct cgc ggc atg ggt gaa tcc aac ccg gtt    912
   Pro Ala Gly Lys Ile Ser Ala Arg Gly Met Gly Glu Ser Asn Pro Val
       290                 295                 300
   act ggc aac acc tgt gac aac gtg aaa gct cgc gct gcc ctg atc gat    960
   Thr Gly Asn Thr Cys Asp Asn Val Lys Ala Arg Ala Ala Leu Ile Asp
   305                 310                 315                 320
   tgc ctg gct ccg gat cgt cgt gta gag atc gaa gtt aaa ggc tac aaa    1008
   Cys Leu Ala Pro Asp Arg Arg Val Glu Ile Glu Val Lys Gly Tyr Lys
                   325                 330                 335
   gaa gtt gta act cag ccg gcg ggt taa 1035
   Glu Val Val Thr Gln Pro Ala Gly
   340

<210> 2
<211> 344
<212> PRT
<213> Klebsiella pneumoniae
<400> 2
   Met Lys Ala Ile Phe Val Leu Asn Ala Ala Pro Lys Asp Asn Thr Trp
   1               5                   10                  15
   Tyr Ala Gly Gly Lys Leu Gly Trp Ser Gln Tyr His Asp Thr Gly Phe
               20                  25                  30
   Tyr Gly Asn Gly Phe Gln Asn Asn Asn Gly Pro Thr Arg Asn Asp Gln
           35                  40                  45
   Leu Gly Ala Gly Ala Phe Gly Gly Tyr Gln Val Asn Pro Tyr Leu Gly
       50                  55                  60
   Phe Glu Met Gly Tyr Asp Trp Leu Gly Arg Met Ala Tyr Lys Gly Ser
   65                  70                  75                  80
   Val Asp Asn Gly Ala Phe Lys Ala Gln Gly Val Gln Leu Thr Ala Lys
                   85                  90                  95
   Leu Gly Tyr Pro Ile Thr Asp Asp Leu Asp Ile Tyr Thr Arg Leu Gly
               100                 105                 110
   Gly Met Val Trp Arg Ala Asp Ser Lys Gly Asn Tyr Ala Ser Thr Gly
           115                 120                 125
   Val Ser Arg Ser Glu His Asp Thr Gly Val Ser Pro Val Phe Ala Gly
       130                 135                 140
   Gly Val Glu Trp Ala Val Thr Arg Asp Ile Ala Thr Arg Leu Glu Tyr
   145                 150                 155                 160
   Gln Trp Val Asn Asn Ile Gly Asp Ala Gly Thr Val Gly Thr Arg Pro
                   165                 170                 175
   Asp Asn Gly Met Leu Ser Leu Gly Val Ser Tyr Arg Phe Gly Gln Glu
               180                 185                 190
   Asp Ala Ala Pro Val Val Ala Pro Ala Pro Ala Pro Ala Pro Glu Val
           195                 200                 205
   Ala Thr Lys His Phe Thr Leu Lys Ser Asp Val Leu Phe Asn Phe Asn
       210                 215                 220
   Lys Ala Thr Leu Lys Pro Glu Gly Gln Gln Ala Leu Asp Gln Leu Tyr
   225                 230                 235                 240
   Thr Gln Leu Ser Asn Met Asp Pro Lys Asp Gly Ser Ala Val Val Leu
                   245                 250                 255
   Gly Tyr Thr Asp Arg Ile Gly Ser Glu Ala Tyr Asn Gln Gln Leu Ser
   260 265 270
   Glu Lys Arg Ala Gln Ser Val Val Asp Tyr Leu Val Ala Lys Gly Ile
   275 280 285
   Pro Ala Gly Lys Ile Ser Ala Arg Gly Met Gly Glu Ser Asn Pro Val
   290 295 300
   Thr Gly Asn Thr Cys Asp Asn Val Lys Ala Arg Ala Ala Leu Ile Asp
   305 310 315 320
   Cys Leu Ala Pro Asp Arg Arg Val Glu Ile Glu Val Lys Gly Tyr Lys
   325 330 335
   Glu Val Val Thr Gln Pro Ala Gly
   340

## Description

L'invention concerne l'utilisation d'une protéine OmpA d'entérobactérie, de préférence la protéine P40 Klebsiella *pneumoniae,* en association avec une substance biologiquement active dont l'efficacité est modulée et/ou liée à l'internalisation de l'OmpA dans les cellules présentatrices d'antigènes, notamment les cellules dendritiques humaines pour la préparation d'une composition pharmaceutique destinée à la prévention et/ou le traitement de maladies, notamment les cancers associés à un antigène tumoral, les maladies auto-immunes ou les maladies infectieuses.

La vaccination est un moyen efficace de prévenir ou réduire les infections virales ou bactériennes. Le succès des campagnes de vaccination dans ce domaine a permis d'étendre le concept de vaccin dans d'autres domaines tels que celui du cancer et des maladies auto-immunes. En ce qui concerne par exemple certaines formes de cancer, l'inefficacité de thérapies classiques et/ou leurs effets secondaires, comme la chimio- ou la radiothérapie, a motivé la recherche de thérapie alternative. Ainsi, les antigènes tumoraux spécifiques exprimés à la surface des cellules tumorales peuvent être utilisés comme cible en immunothérapie pour l'élimination de ces cellules. Un des problèmes majeurs couramment rencontrés pour la préparation de ces vaccins est que les antigènes vaccinaux lorsqu'ils sont administrés seuls chez l'hôte, ne sont pas assez immunogéniques pour induire une réponse immunitaire suffisamment efficace pour conférer la protection recherchée. Ces antigènes sont ainsi souvent couplés de manière covalente à une molécule porteuse telle que par exemple épitope de la toxine diphtérique, l'anatoxine tétanique (TT), un antigène de surface du virus de l'hépatite B, l'antigène VP1 du virus de la poliomyélite ou tout autre toxine ou antigène viral ou bactérien tel que des protéines antigéniques issues de la membrane externe d'entérobactérie qui ont la propriété de potentialiser la réponse immunitaire (humorale et cellulaire) de l'antigène qui lui est associée comme la protéine OmpA nommée P40 issue de Klebsiella *pneumoniae* (décrite dans les demandes internationales de brevet WO 95/27787 et W0 96/14415). Néanmoins, dans la plupart des cas un autre composant s'est avéré nécessaire pour augmenter l'efficacité du vaccin, et actuellement le seul adjuvant autorisé chez l'homme est l'Alum.

Grâce à l'immunologie, il a été récemment découvert que les cellules dendritiques (CD) jouaient un rôle majeur dans le système immunitaire. Ces cellules, dérivées des cellules souches de la moelle osseuse, sont des cellules présentatrices d'antigènes professionnelles impliquées dans la réponse immune primaire spécifique d'un antigène (Peters J. et al., 1996). Elles ingèrent ou intemalisent les antigènes et présentent les fragments de ces antigènes à des cellules T naives. Cette ingestion induit à la surface des cellules dendritiques l'expression de molécules de costimulation telles le CD80 et le CD86. Ces molécules permettent une interaction étroite avec les cellules T (Girolomoni G. et Ricciardi-Castagnoli P., 1997, Immunol. Today, 18, 102-104). Les cellules dendritiques sont distribuées de manière diffuse dans les tissus. Elles sont retrouvées aux niveaux de la peau et des organes lymphoïdes (Hinrich J. et al., 1996, Immunol. Today, 17, 273-277).

Grâce à leur efficacité à présenter les antigènes et à stimuler le système immunitaire, les cellules dendritiques ont été utilisées pour générer des réponses cytotoxiques CTL antivirales (Ludewig B. et al., 1998, J. Virol., 72, 3812-3818 ; Brossard P. et al., 1997, J. Immunol., 158, 3270-3276) ou anticancéreuses (Nestle F.O. et al., 1998, Nat. Med., 4, 328-332). Les approches ont consisté à charger les cellules dendritiques *ex vivo* avec l'antigène d'intérêt (peptides ou lysat cellulaire) et réimplanter ces cellules chez le patient. D'autres approches consistent à transfecter *ex vivo* les cellules dendritiques avec le gène codant pour l'antigène d'intérêt et à réinjecter ces cellules transfectées (Gilboa E. et al., 1998, Cancer Immunol. Immunother., 46, 82-87). Ces approches ont été utilisées avec succès chez la souris et récemment chez l'homme (Hsu F.J. et al., 1996, Nat. Med., 2, 52-58). Les cellules dendritiques chargées en antigènes présentent les peptides par le biais de molécules de classe I ou II et induisent l'activation des lymphocytes T CD4 ou CD8+. Par conséquent, la possibilité de diriger les antigènes désignés, tels que des protéines ou des polysaccharides, ou des vecteurs viraux capables de transférer des gènes codant pour ces antigènes vers les cellules dendritiques permettrait d'améliorer l'efficacité de la stimulation du système immunitaire. De plus, le ciblage spécifique des cellules présentatrices d'antigènes (CPA), notamment les cellules dendritiques, permettrait d'éviter les étapes de prélèvement, de purification, de traitement *ex vivo* de cellules CPA autologues ou hétérologues avec les antigènes tumoraux ou les vecteurs viraux et la réimplantation des cellules CPA traitées.

Afin de cibler spécifiquement les cellules dendritiques avec des substances actives d'intérêt, telles que des protéines ou des vecteurs viraux capables de transférer des gènes codant pour ces protéines d'intérêt, de nombreux travaux ont consisté à identifier des molécules qui se fixeraient préférentiellement sur les cellules dendritiques ou des récepteurs qui seraient exprimés spécifiquement sur les cellules dendritiques. Un récepteur DEC 205 impliqué dans la prise en charge de l'antigène a été identifié sur des cellules dendritiques murines (Jiang W. et al., 1995, Nature, 375, 151-155) et humaines (Kato M. et al., 1998, Immunogenetics, 47, 442-450). L'analyse de la structure de ce récepteur met en évidence des domaines de reconnaissance d'hydrates de carbone qui seraient impliqués dans la capture, l'intemalisation et/ou la présentation d'antigènes portant des résidus d'hydrates de carbone. Néanmoins, les auteurs ne donnent aucune information sur les ligands pouvant être fixés par ce récepteur. D'autre part, les auteurs mentionnent que les domaines de reconnaissance d'hydrates de carbone du récepteur DEC-205 qui seraient impliqués dans la capture, l'internalisation et/ou la présentation d'antigènes (domaines riches en cystéine) sont également présents dans plus de 50 protéines dont certains récepteurs cellulaires.

On peut également citer la demande de brevet publiée sous le numéro WO 96/14415 suggérant que l'effet adjuvant de la protéine OmpA P40 de Klebsiella *pneumoniae* était lié à la reconnaissance spécifique de ses séquences par des cellules présentatrices d'antigènes et permettrait de cibler des antigènes vers ces cellules.

Ainsi, il existe aujourd'hui un besoin de disposer d'un composé capable de cibler spécifiquement une cellule présentatrice d'antigène (CPA), notamment une cellule dendritique, et qui soit capable en outre d'être internalisé par ladite cellule. Un tel composé capable de se fixer spécifiquement sur ces cellules, puis d'être internalisé aurait comme avantage de pouvoir être utilisé comme composé pour le transport et le ciblage de substance biologiquement active dont l'efficacité est modulée et/ou liée à la fixation et/ou à l'intemalisation de cette substance par ces cellules. D'autre part, il serait avantageux que ce composé recherché puisse être facilement associé à la substance active par couplage chimique, par couplage résultant d'une fusion génétique ou puisse être exprimé à la surface d'une cellule hôte ou encore à la surface d'une particule virale pour le transfert de gène d'intérêt dans ces cellules CPA.

Les auteurs de la présente invention ont mis en évidence de manière surprenante qu'une protéine de la membrane externe de type OmpA d'entérobactérie, notamment la protéine P40 de Klebsiella *pneumoniae*, est capable non seulement de se fixer spécifiquement sur une cellule CPA mais également capable d'être intemalisée par ladite cellule CPA, notamment par une cellule dendritique.

Ainsi, la présente invention est relative à l'utilisation d'une protéine OmpA d'entérobactérie ou d'un de ses fragments en association avec une substance biologiquement active dont l'efficacité est modulée et/ou liée à l'internalisation de l'OmpA dans les cellules présentatrices d'antigènes pour la préparation d'une composition destinée à prévenir ou à traiter les cancers, de préférence les cancers associés à un antigène tumoral, les maladies auto-immunes, les allergies, les rejets de greffes, les maladies cardiovasculaires, les maladies du système nerveux central, les maladies inflammatoires, les maladies infectieuses ou les maladies liées à une immunodéficience.

Par cellules présentatrices d'antigènes, on entendra désigner dans la présente invention, les cellules CPA professionnelles formant partie intégrante du système immunitaire telles que les cellules dendritiques, les macrophages, les lymphocytes B ou les monocytes.

Dans la présente invention, on entendra désigner également par le terme « protéine » les peptides ou les polypeptides et par le terme « OmpA » (pour « Outer Membrane Protéin »), les protéines de la membrane externe de type A.

Par fragment d'une protéine OmpA, on entend désigner tout fragment de séquence d'acides aminés compris dans la séquence d'acides aminés de la protéine OmpA capable de se fixer spécifiquement sur les cellules CPA, notamment les cellules dendritiques, et comprenant au moins 5 acides aminés, de préférence 10 acides aminés ou de manière plus préférée 15 acides aminés, lesdits fragments étant en outre capables d'être intemalisés dans lesdites cellules CPA.

Par « substance biologiquement active », on entend désigner tout composé capable d'exercer une activité thérapeutique et dont l'activité peut être modulée par l'intermédiaire des cellules CPA. On peut citer comme exemple de telles substances biologiquement actives, mais sans s'y limiter, les composés immunogènes tels que des antigènes ou haptènes de nature protéique, poly- ou oligosaccharidique, glycoprotéique, lipoprotéique ou en général d'origine organique, ces composés immunogènes pouvant être portés par des structures complexes telles que des bactéries ou des particules virales.

Par « substance biologiquement active », on entend également désigner tout composé capable de modifier l'activité fonctionnelle des cellules CPA, en particulier leur croissance, leur différentiation ou leur système d'expression. On peut citer comme exemple de telles substances biologiquement actives, mais sans s'y limiter, les facteurs de croissance cellulaire dont les cytokines (IL-4, IL-3, GM-CSF, TNF-α), les acides nucléiques codant pour des protéines d'intérêt homologues ou hétérologues et capables d'être exprimées par les cellules CPA.

L'invention a également pour objet l'utilisation d'une protéine OmpA d'entérobactérie ou d'un de ses fragments selon l'invention, caractérisée en ce que ladite protéine OmpA d'entérobactérie ou l'un de ses fragments se fixe spécifiquement sur les cellules présentatrices d'antigènes et en ce que ladite protéine OmpA d'entérobactérie ou l'un de ses fragments est internalisée dans les cellules présentatrices d'antigènes.

De préférence, l'invention comprend l'utilisation dune protéine OmpA d'entérobactérie ou d'un de ses fragments selon l'invention, caractérisée en ce que lesdites cellules présentatrices d'antigènes sont choisies parmi les cellules dendritiques, les monocytes ou les lymphocytes B, de manière plus préférée, les cellules dendritiques.

Dans un mode de réalisation particulier, l'invention comprend l'utilisation d'une protéine OmpA d'entérobactérie ou d'un de ses fragments selon l'invention, caractérisée en ce que ladite protéine OmpA d'entérobactérie, ou l'un de ses fragments, est obtenue à partir d'une culture de ladite entérobactérie par un procédé d'extraction.

Les procédés d'extraction de protéines de membrane bactériennes sont connus de l'homme de l'art et ne seront développés dans la présente description. On peut citer par exemple, mais sans s'y limiter le procédé d'extraction décrit par Haeuw J.H. et al. (Eur. J. Biochem, 255, 446-454, 1998).

Dans un autre mode de réalisation préféré, l'invention comprend également l'utilisation d'une protéine OmpA d'entérobactérie ou d'un de ses fragments selon l'invention, caractérisée en ce que ladite protéine OmpA d'entérobactérie, ou l'un de ses fragments, est obtenue par voie recombinante.

Les méthodes de préparation de protéines recombinantes sont aujourd'hui bien connues de l'homme de l'art et ne seront pas développées dans la présente description, on pourra néanmoins se référer à la méthode décrite dans les exemples. Parmi les cellules utilisables pour la production de ces protéines recombinantes, il faut citer bien entendu les cellules bactériennes (Olins P.O. et Lee S.C., 1993, Recent advances in heterologous gene expression in E. coli. Curr. Op. Biotechnology 4:520-525), mais également les cellules de levure (Buckholz R.G., 1993, Yeast Systems for the Expression of Heterologous Gene Products. Curr. Op. Biotechnology 4:538-542), de même que les cellules animales, en particulier les cultures de cellules de mammifère (Edwards C.P. et Aruffo A., 1993, Current applications of COS cell based transient expression systems. Curr. Op. Biotechnology 4:558-563) mais également les cellules d'insectes dans lesquelles on peut utiliser des procédés mettant en oeuvre des baculovirus par exemple (Luckow V.A., 1993, Baculovirus systems for the expression of human gene products. Curr. Op. Biotechnology 4:564-572).

De manière tout à fait préférée, l'utilisation selon l'invention est caractérisée en ce que ladite entérobactérie est Klebsiella *pneumoniae*.

En particulier, l'invention est relative à l'utilisation selon l'invention, caractérisée en ce que la séquence d'acides aminés de ladite protéine OmpA de Klebsiella *pneumoniae*, ou l'un de ses fragments, comprend :
a) la séquence d'acides aminés de séquence SEQ ID N° 2 ;
b) la séquence d'acides aminés d'une séquence présentant un degré d'identité après alignement optimal d'au moins 80 %, de préférence d'au moins 85 %, 90 % ou 95 % avec la séquence SEQ ID N° 2 ; ou
c) la séquence d'acides aminés d'un fragment d'au moins 5 acides aminés d'une séquence SEQ ID N° 2 capable de se fixer spécifiquement sur les cellules présentatrices d'antigènes, lesdits fragments étant en outre capables d'être intemalisés dans lesdites cellules présentatrices d'antigènes.

Par séquence présentant une homologie d'au moins 80 %, de préférence d'au moins 85 %, 90 % ou 95 % avec la séquence de référence SEQ ID N° 2, on entend désigner une séquence d'acides aminés présentant un degré d'identité après alignement optimal respectivement d'au moins 80 %, 85 %, 90 % ou 95 % avec la séquence de référence SEQ ID N° 2, ladite séquence homologue ou l'un de sesdits fragments d'au moins 5 acides aminés tels que définis précédemment en c) étant caractérisés en ce qu'ils se fixent spécifiquement sur les cellules présentatrices d'antigènes et, le cas échéant, en ce qu'ils sont internalisés dans les cellules présentatrices d'antigènes.

Par « pourcentage d'identité » entre deux séquences d'acide nucléique ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. Le meilleur alignement ou alignement optimal est l'alignement pour lequel le pourcentage d'identité entre les deux séquences à comparer, comme calculé ci-après, est le plus élevé. Les comparaisons de séquences entre deux séquences d'acide nucléique ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, Wl, ou encore BLASTN ou BLASTX, Altschul et al., J. Mol. Biol. 215, 403, 1990).

Le pourcentage d'identité entre deux séquences d'acide nucléique ou d'acides aminés est déterminé en comparant ces deux séquences alignées de manière optimale par fenêtre de comparaison dans laquelle la région de la séquence d'acide nucléique ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

L'invention comprend en outre l'utilisation selon l'invention, caractérisée en ce que ladite substance biologiquement active est choisie parmi les protéines ou les peptides, les lipopeptides, les polysaccharides, les oligosaccharides, les acides nucléiques, les lipides et les substances chimiques.

La présente invention a aussi pour objet l'utilisation d'une protéine OmpA d'entérobactérie ou d'un de ses fragments selon l'invention, caractérisée en ce que ladite substance biologiquement active est couplée par liaison covalente avec ladite protéine OmpA ou l'un de ses fragments, notamment par un couplage chimique.

Dans un mode de réalisation particulier, l'utilisation selon l'invention est caractérisée en ce qu'il est introduit un ou plusieurs éléments de liaison dans ladite protéine OmpA, ou l'un de ses fragments, et/ou de ladite substance biologiquement active pour faciliter le couplage chimique, de préférence ledit élément de liaison introduit est un acide aminé.

Selon l'invention, il est possible d'introduire un ou plusieurs éléments de liaison, notamment des acides aminés pour faciliter les réactions de couplage entre la protéine OmpA, ou l'un de ses fragments, et la substance biologiquement active, telle qu'un antigène ou un haptène. Le couplage covalent entre la protéine OmpA, ou l'un de ses fragments, et la substance biologiquement active, telle qu'un antigène ou un haptène selon l'invention peuvent être réalisés à l'extrémité N- ou C- terminale de la protéine OmpA, ou l'un de ses fragments. Les réactifs bifonctionnels permettant ce couplage seront déterminés en fonction de l'extrémité de la protéine OmpA, ou l'un de ses fragments, choisie pour effectuer le couplage et de la nature de la substance biologiquement active à coupler.

Dans un autre mode de réalisation particulier, l'utilisation selon l'invention est caractérisée en ce que ladite substance biologiquement active couplée par liaison covalente avec ladite protéine OmpA, ou l'un de ses fragments, est une protéine chimérique recombinante résultante de l'expression d'une construction d'acide nucléique codant pour ladite substance biologiquement active et pour ladite protéine OmpA, ou l'un de ses fragments.

Les conjugués issus d'un couplage à ladite protéine OmpA, ou l'un de ses fragments, peuvent être préparés par recombinaison génétique. La protéine chimérique ou hybride (conjugué) peut être produite par des techniques d'ADN recombinant par insertion ou addition à la séquence d'ADN codant pour ladite protéine OmpA, ou l'un de ses fragments, d'une séquence codant pour ladite substance biologiquement active de nature protéique.

Les procédés de synthèse des molécules hybrides englobent les méthodes utilisées en génie génétique pour construire des polynucléotides hybrides codant pour les séquences polypeptidiques recherchées. On pourra, par exemple, se référer avantageusement à la technique d'obtention de gènes codant pour des protéines de fusion décrite par D.V. Goeddel (Gene expression technology, Methods in Enzymology, vol. 185, 3-187, 1990).

L'invention concerne tout particulièrement l'utilisation d'une protéine OmpA d'entérobactérie ou d'un de ses fragments selon l'invention, caractérisée en ce que ladite substance biologiquement active est un antigène ou un haptène.

Sous un autre aspect, l'invention concerne l'utilisation d'une protéine OmpA d'entérobactérie ou d'un de ses fragments selon l'invention pour moduler la réponse immune vis-à-vis d'un antigène ou d'un haptène, de préférence pour améliorer la réponse immune vis-à-vis d'un antigène ou d'un haptène.

L'invention comprend également l'utilisation d'une protéine OmpA d'entérobactérie ou d'un de ses fragments selon l'invention pour la préparation d'une composition pharmaceutique destinée à prévenir ou à traiter une maladie par une substance active dont l'efficacité est modulée et/ou liée à son intemalisation par des cellules présentatrices d'antigènes, de préférence par des cellules dendritiques.

L'invention a en particulier pour objet l'utilisation d'une protéine OmpA d'entérobactérie ou d'un de ses fragments selon l'invention, pour la préparation d'une composition pharmaceutique vaccinale destinée à prévenir ou à traiter une maladie infectieuse ou un cancer associé à un antigène tumoral.

L'invention comprend en outre l'utilisation selon l'invention, caractérisée en ce que ladite composition pharmaceutique comprend en outre un adjuvant favorisant la réponse immune, tel que l'Alum.

L'invention comprend également l'utilisation selon l'invention, caractérisée en ce que ladite composition pharmaceutique est véhiculée sous une forme permettant d'améliorer sa stabilité et/ou son immunogénicité, notamment sous la forme d'un liposome. L'invention comprend également l'utilisation d'une cellule hôte transformée capable d'exprimer une protéine chimérique recombinante résultante de l'expression d'une construction d'acide nucléique codant une protéine OmpA d'entérobactérie ou l'un de ses fragments et une substance biologiquement active consistant en un peptide et dont l'efficacité est modulée et/ou liée à l'internalisation de l'OmpA dans les cellules présentatrices d'antigènes pour la préparation d'une composition pharmaceutique destinée à prévenir ou à traiter les cancers, de préférence les cancers associés à un antigène tumoral, les maladies auto-immunes, les allergies, les rejets de greffes, les maladies cardiovasculaires, les maladies du système nerveux central, les maladies inflammatoires, les maladies infectieuses ou les maladies liées à une immunodéficience.

Les légendes des figures et exemples qui suivent sont destinés à illustrer l'invention sans aucunement en limiter la portée.

### Légendes des figures :

Figure 1 : Fixation de rP40-Alexa sur différents types cellulaires.
   Après incubation de rP40-Alexa sur différents types cellulaires, la fixation spécifique de rP40-Alexa (trait gras) est mesurée par cytométrie en flux. La fixation d'une protéine non relevante (glycophorine) est représentée en trait fin.
Figure 2 : Influence de la concentration de rP40 sur la fixation sur les cellules dendritiques.
Figure 3 : Inhibition de la fixation de rP40-Alexa par rP40 non marquée sur des cellules dendritiques.
   Après incubation de cellules dendritiques avec différentes concentrations de rP40 non marquée, rP40-Alexa est ajoutée. La fixation de rP40-Alexa est quantifiée par cytométrie en flux.
Figure 4 : Evaluation de la fixation de différentes protéines marquées sur les cellules dendritiques.
   Des protéines porteuses P40, TT (anatoxique tétanique) et BB (dérivées de la protéine G du streptocoque) marquées à l'Alexa sont incubées avec des cellules dendritiques (trait plein). Une protéine non relevante est utilisée comme témoin négatif (trait fin). La fixation est mesurée par cytométrie en flux.

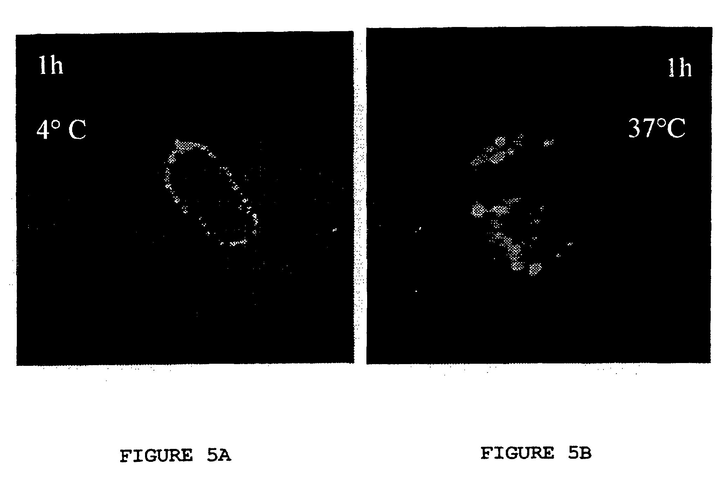

Figures 5A et 5B : Internalisation de rP40-Alexa dans les cellules dendritiques.
   Après incubation de cellules dendritiques avec rP40-Alexa à 4°C (panel gauche, figure 5A) ou à 37°C (panel droit, figure 5B), les cellules sont observées par microscopie confocale (grossissement x 220).

### Exemple 1 : Clonage du gène rP40

Le gène codant pour la protéine recombinante P40 nommée rP40 a été obtenu par amplification par PCR à partir de l'ADN génomique de Klebsiella *pneumoniae* IP 1145 (Nguyen et col., Gene, 1998). Le fragment de gène codant de rP40 est inséré dans divers vecteurs d'expression, en particulier un vecteur sous le contrôle du promoteur de l'opéron Trp. La séquence d'acides aminés de la protéine rP40 et la séquence nucléotidique codant pour la protéine P40 sont représentées respectivement par les séquences SEQ ID N° 2 et SEQ ID N° 1 dans la liste des séquences ci-après.

Une souche productrice E. *coli* K12, a été transformée par un vecteur d'expression pvaLP40. La protéine rP40 est produite sous forme de corps d'inclusion avec un rendement important (> 10 %, g de protéines/g de biomasse sèche). Cet exemple n'est qu'une illustration de l'expression de la rP40, mais elle peut être étendue à d'autres souches bactériennes ainsi que d'autres vecteurs d'expression.

### Exemple 2 : Procédé de fermentation de protéines de fusion rP40

Un erlenmeyer contenant 250 ml de milieu TSB (Tryptic Soy Broth, Difco) renfermant de l'Ampicilline (100 µg/ml, Sigma) et de la Tétracycline (8 µg/ml, Sigma) est inoculé avec la souche E. *coli* recombinante décrite ci-dessus. L'incubation est réalisée pendant une nuit à 37°C puis 200 ml de cette culture est utilisée pour ensemencer 2 litres de milieu de culture dans un fermenteur (Biolafitte, France). De manière assez classique, le milieu de culture peut être composé d'agents chimiques, supplémentés par les vitamines, des extraits de levure, connus pour avoir une croissance à densité élevée de cellules bactériennes.

Les paramètres contrôlés durant la fermentation sont : le pH, l'agitation, la température, le taux d'oxygénation, l'alimentation de sources combinées (Glycérol ou Glucose). De manière générale, le pH est régulé à 7,0, la température est fixée à 37°C. La croissance est contrôlée en alimentant en glycérol (87 %) à un débit constant (12 ml/h) pour maintenir le signal de tension de, l'oxygène dissous à 30 %. Lorsque la turbidité de la culture (mesurée à 580 nm) atteint la valeur de 80 (après environ 24 heures de culture), la production des protéines est déclenchée par addition de l'acide indole acrylique (IAA) à la concentration finale de 25 mg/l. Environ 4 heures après induction, les cellules sont récoltées par centrifugation. La quantité de biomasse humide obtenue est d'environ 200 g.

### Exemple 3 : Procédé d'extraction et de purification de la protéine rP40

### Extraction de la rP40

Après centrifugation du bouillon de culture (4000 rpm, 10 min, 4°C), les cellules sont remises en suspension dans un tampon Tris-HCl 25 mM pH 8,5. Les insolubles ou corps d'inclusion sont obtenus après un traitement par le lysozyme (0,5 g/litre, 1 heure température ambiante / agitation douce). Le culot de corps d'inclusion obtenu par centrifugation (15 min à 10 000 g à 4°C) est repris dans un tampon Tris-HCl 25 mM à pH 8,5 et 5 mM MgCl2, puis centrifugé (15 min à 10 000 g).

On solubilise les corps d'inclusion à 37°C pendant 2 heures dans un tampon Tris-HCl 25 mM pH 8,5 contenant 7 M urée (agent dénaturant) et 10 mM Dithiothréitol (réduction des ponts disulfures). Une centrifugation (15 min à 10 000 g) permet d'éliminer les particules non solubles.

On resuspend ensuite dans 13 volumes de tampon Tris-HCl 25 mM pH 8,5 contenant du NaCl (8,76 g/l) et du Zwittergent 3-14 (0,1 %, p/v). La solution est laissée pendant une nuit à température ambiante sous agitation douce au contact de l'air (favoriser la renaturation de la protéine par dilution et réoxydation des ponts disulfures).

### Purification de la protéine rP40

### Etape de chromatographie d'échange d'anions.

Après une nouvelle centrifugation, la solution est dialysée contre un tampon Tris-HCI 25 mM pH 8,5 contenant 0,1 % Zwittergent 3-14 (100 X volumes de tampon) pendant une nuit à 4°C.

Le dialysat est déposé sur une colonne contenant un support de type échangeur d'anions forts (gel Biorad Macro Prep High Q) équilibrée dans le tampon décrit ci-dessus à un débit linéaire de 15 cm/h. Les protéines sont détectées à 280 nm. La protéine rP40 est éluée, avec un débit linéaire de 60 cm/h, pour une concentration de 0,2 M en NaCl dans le tampon TrisHCl 25 mM, pH 8,5 ; 0,1 % Zwittergent 3-14.

### Etape de chromatographie d'échange de cations

Les fractions contenant la protéine rP40 sont poolées et concentrées par ultrafiltration à l'aide d'un système de cellule à agitation Amicon utilisé avec une membrane Diaflo de type YM10 (seuil de coupure 10 kDa) pour des volumes de l'ordre de 100 ml, ou à l'aide d'un système de filtration à flux tangentiel Minitan Millipore utilisé avec des plaques de membranes possédant un seuil de coupure 10 kDa pour des volumes supérieurs. La fraction ainsi concentrée est dialysée pendant une nuit à 4°C contre un tampon citrate 20 mM pH 3,0, à 0,1 % de Zwittergent 3-14.

Le dialysat est déposé sur une colonne contenant un support de type échangeur de cations forts (gel Biorad Macro Prep High S) équilibrée dans le tampon citrate 20 mM pH 3,0, à 0,1 % de Zwittergent 3-14. La protéine rP40 est éluée (vitesse 61 cm/h) pour une concentration 0,7 M en NaCl. Les profils étectrophorétiques montrent un degré de pureté de l'ordre de 95 %. L'état de la protéine est suivi par SDS-PAGE. Selon sa forme dénaturée ou native, la protéine P40 extraite de la membrane de Klebsiella *pneumoniae* possède un comportement électrophorétique (migration) caractéristique. La forme native (structure en feuillets p) présente en effet une masse moléculaire plus faible que la forme dénaturée (structure en hélices α) sous l'action d'un agent dénaturant, tel que l'urée ou le chlorhydrate de guanidine, ou par chauffage à 100°C en présence de SDS). La protéine rP40 n'est pas correctement renaturée en fin de renaturation, que celle-ci soit réalisée en absence ou en présence de 0,1 % ; (p/v) Zwittergent 3-14. Par contre, une renaturation totale est obtenue après dialyse contre un tampon Tris/HCl 25 mM pH 8,5 contenant 0,1 % (p/v) de Zwittergent 3-14. Toutefois, il faut noter que cette renaturation n'est obtenue que lorsque l'étape de dilution et le traitement à température ambiante sont réalisés eux-mêmes en présence de Zwittergent 3-14 (résultats négatifs en absence de détergent).

### Exemple 4 : Fixation spécifique de rP40 sur les cellules présentatrices d'antigène (CPA). Méthodologie

### Purification des lymphocytes T humains

Les cellules mononucléées (CMN) sont isolées à partir du sang périphérique de volontaires sains par centrifugation (1800 rpm, 20 min, température ambiante), sur un gradient de Ficoll. Après centrifugation, les CMN, situées à l'interface ficoll/plasma, sont récoltées et lavées 2 fois avec du milieu de culture complet (MC) (RPMI 1640 + 10 % SVF + L-glutamine + antibiotique). Les lymphocytes T sont alors isolés par la technique du rosetting qui utilise leur capacité à se fixer aux globules rouges de mouton (GRM). Brièvement, les CMN sont incubées avec des GRM pendant 1 heure à 4°C. Après centrifugation sur gradient de ficoll, les lymphocytes B et les monocytes sont situés à l'interface alors que les lymphocytes T fixés aux GRM sont dans le culot cellulaire. Après récupération du culot cellulaire et lyse des GRM avec une solution saline hypotonique, la pureté des lymphocytes T est appréciée par cytométrie en flux avec un anticorps anti-CD3 et est supérieure à 95 %.

### Purification des monocytes humains

Les monocytes sont purifiés à partir des CMN par sélection positive en utilisant la technologie MACS (Magnetic Activated Cetl Sorter). Les CMN sont marquées avec un anticorps anti-CD14 couplé à des particules magnétiques puis passées sur une colonne aimantée. Les monocytes sur lesquels sont fixés les complexes anticorps-colloïde restent dans la colonne alors que les cellules n'ayant pas fixé l'anticorps sont éluées par lavages successifs. Ensuite les monocytes sont décrochés en effectuant des lavages en absence d'aimant. La pureté de la fraction collectée est supérieure à 98%.

### Génération de cellules dendritiques humaines (CD) à partir de monocytes

Les monocytes purifiés sont cultivés à la concentration de 106/ml dans du MC pendant 6 à 7 jours en présence de IL 4 (20 ng/ml) et de GMCSF (20 ng/ml). Les CD générées à ce stade sont des CD immatures qui expriment le CDIa et pas ou peu le CD83. Leur phénotype est vérifié par la technique de cytométrie en flux.

### Purification des lymphocytes B humains à partir d'amygdales

Les amygdales sont broyées et les cellules récoltées sont déposées sur un gradient de ficoll. Les CMN recueillies à l'interface sont lavées puis incubées avec des GRM. Après ficoll, les lymphocytes B sont situés à l'interface alors que les lymphocytes T fixés aux GRM sont dans le culot cellulaire. Les lymphocytes B sont alors lavés. Leur pureté, vérifiée par cytométrie en flux, est supérieure à 96%.

### Culture des lignées cellulaires

Les lignées cellulaires RPMI 8866, DAUDI, HL60 et Jurkat sont cultivées dans du MC.

### Couplage de rP40 au fluorochrome Alexa488

La concentration de la protéine rP40 est ajustée à 2 mg/ml dans du PBS. A 500 µl de la protéine sont ajoutés 50 µl de bicarbonate de sodium à 1 M. La solution est alors transférée dans un tube de réaction contenant le colorant Alexa488 et le couplage s'effectue à température ambiante. Après 1 h, la réaction de couplage est arrêtée par ajout de 15 µl d'hydroxylamine. La protéine marquée est séparée du colorant libre par purification sur colonne.

La quantité de rP40 marquée à l'Alexa488 est ensuite estimée par dosage colorimétrique.

### - Etude de la fixation de P40-Alexa488 sur les différentes cellules par cytométrie en flux.

Pour chaque marquage, 200 000 cellules sont lavées avec du tampon FACS (PBS + 1 % BSA + 0,01 % azide de sodium) et resuspendues, dans une plaque 96 puits à fond conique, dans 50 µl de tampon FACS. La protéine P40-Alexa488 ou la protéine contrôle (glycophorine-Alexa488) sont alors ajoutées à 10⁻⁶M pendant environ 1 heure à 4°C. Après incubation, les cellules sont alors lavées 3 fois avec du tampon FACS puis resuspendues dans 200 µl de ce même tampon et analysées par cytométrie en flux.

### Résultat

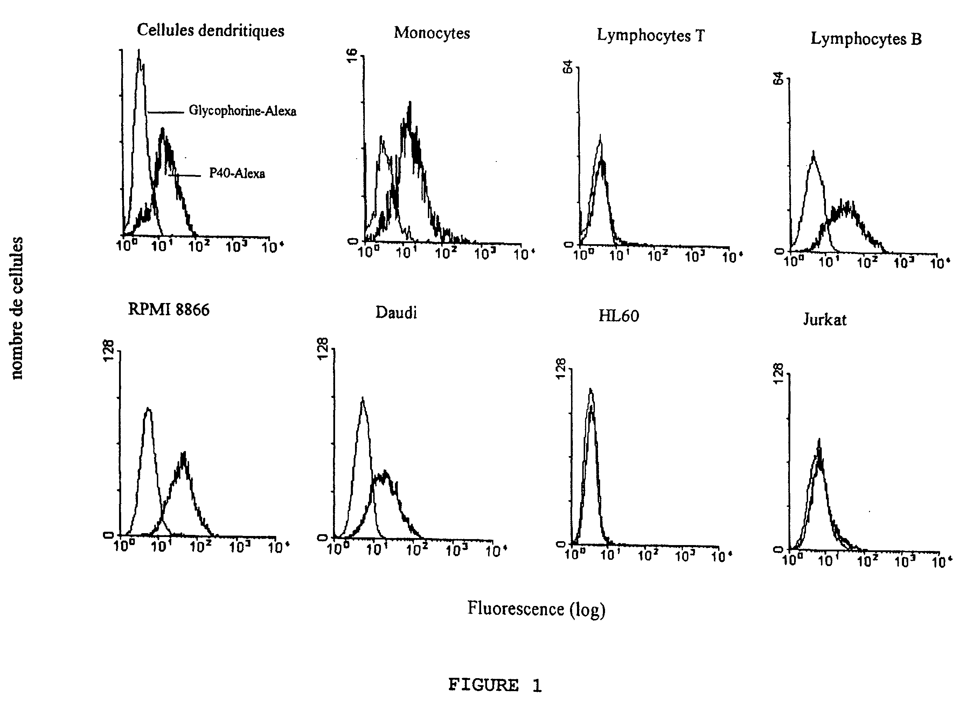

La protéine rP40 se fixe sélectivement sur les CPA humaines telles que :
- les monocytes issus du sang périphérique,
- les cellules dendritiques générées à partir des monocytes du sang périphérique,
- les lymphocytes B issus d'amygdale, les lignées lymphocytaires B : DAUDI et RPMI 8866 (cf. Fig. 1) et les lymphocytes B issus du sang périphérique (résultat non présenté).

Aucune fixation n'est observée sur des cellules qui ne possèdent pas la capacité de présenter des antigènes tels que dés lymphocytes T du sang périphérique non activés, la lignée lymphocytaire T Jurkat non activée et la lignée monocytaire HL60 non activée.

### Exemple 5 : La fixation de rP40 aux CD est spécifique

### 1) La fixation de rP40 aux CD est dépendante de la dose.

### Méthode

200 000 CD sont lavées avec du tampon FACS et incubées dans 50 µl de tampon en présence de différentes concentrations de rP40 (de 10⁻¹⁰ à 5 x 10⁻⁶ M) pendant environ 1 heure à 4°C. Après incubation, les cellules sont lavées 3 fois avec du tampon FACS puis resuspendues dans 50 µl de ce même tampon contenant 5 µg/ml d'un anticorps polyclonal de lapin anti-P40 ou d'un anticorps IgG de lapin contrôle. Après 20 minutes d'incubation, les cellules sont relavées et incubées dans 100 µl de tampon FACS contenant un anticorps polyclonal de chèvre anti-IgG de lapin marqué à la fluorescéine (dilué au 1:200). Après 20 minutes d'incubation, les cellules sont lavées, reprises dans du tampon FACS et analysées par cytométrie en flux.

### Résultat

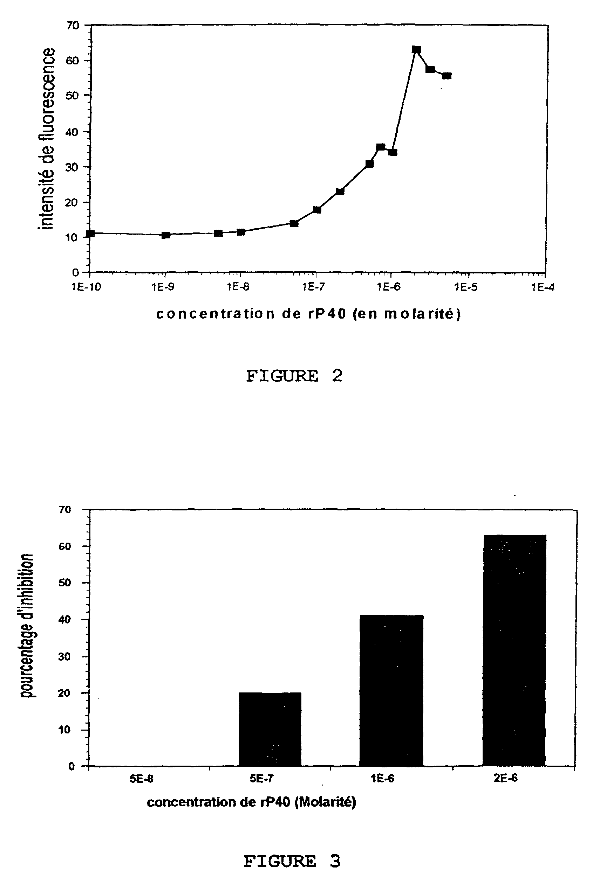

La fixation de rP40 au CD est significative à partir de 10⁻⁷ M (p<0.001) et maximale à 2 x 10⁻⁶ M (cf. Fig. 2).

### 2) La protéine rP40 non marquée diminue la fixation de rP40 Alexa488 aux CD.

### Méthode

Afin de démontrer la spécificité de la fixation de P40, une compétition entre rP40-Alexa488 et rP40 non marquée a été réalisée. Les CD ont été incubées 10 minutes avec 5 x 10⁻⁸ à 2 x 10⁻⁶ M de rP40 non marqué puis P40-Alexa488 (utilisé à 2 × 10⁻⁶ M) a été ajouté. Après 20 minutes d'incubation à 4°C, les cellules ont été analysées par cytométrie en flux comme décrit auparavant.

### Résultat

La protéine rP40 non marquée inhibe de manière dépendante de la dose la fixation de 2 x 10⁻⁶ M de P40 Alexa488 (à plus de 60 % lorsqu'il est utilisé à 2 x 10⁻⁶ M) (cf. Fig. 3).

### Exemple 6 : Parmi les protéines porteuses, TT, BB et rP40, seule la protéine rP40 se fixe aux CD.

### Méthode

Les protéines porteuses, anatoxine tétanique (TT) et BB (d'origine de la protéine G du Streptocoque ayant une affinité à l'albumine humaine), ainsi que la protéine rP40 et la protéine contrôle glycophorine A ont été marquées par Alexa488 comme précédemment décrit. La fixation de ces molécules sur les CD a été évaluée par cytométrie en flux comme décrit auparavant. Brièvement, 200 000 CD sont lavées avec du tampon FACS et incubées dans 50 µl de tampon en présence de 10⁻⁶ M de chacune des protéines marquées par Alexa488 pendant environ 1 heure à 4°C. Après incubation, les cellules sont lavées 3 fois avec du tampon FACS puis resuspendues dans 200 µl de ce même tampon et analysées par cytométrie en flux.

### Résultat

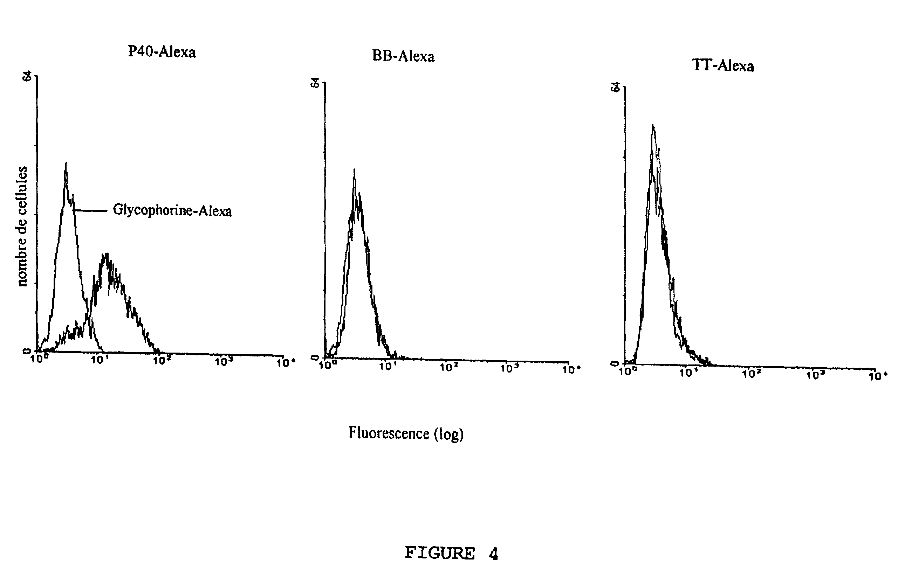

A la concentration de 10⁻⁶ M, seule rP40 se fixe aux cellules dendritiques. Aucune fixation de TT, BB et glycophorine n'est détectée (cf. Fig. 4).

### Exemple 7 : rP40 est intemalisée par les CD

### Méthode

200 000 CD sont lavées avec du tampon PBS-BSA 1 % et resuspendues, dans une plaque 96 puits à fond conique, dans 50 µl de tampon PBS-BSA (tampon phosphate salin - sérum albumine bovine). La protéine rP40-Alexa488 ou la protéine glycophorine-Alexa488 est alors ajoutée à 2 x 10⁻⁶ M. Une cinétique d'intemalisation est effectuée en incubant les cellules avec les protéines marquées à rAlexa, à 37°C, de 15 minutes à 2 heures. Un contrôle négatif d'intemalisation est réalisé dans les mêmes conditions en changeant les paramètres suivants : ajout d'azide de sodium 0,01 % au tampon PBS-BSA et incubation des cellules à 4°C avec les protéines marquées à l'Alexa.

Après incubation, les cellules sont alors lavées 3 fois avec du tampon PBS-BSA, resuspendues dans 100 µl de ce même tampon puis cytocentrifugées sur des lames de microscope. Les lames sont ensuite analysées par microscopie confocale.

### Résultat

L'observation des cellules incubées à 37°C avec rP40-Alexa montre un marquage intracytoplasmique détectable dès 30 minutes et toujours observé après 2 h d'incubation : un résultat représentatif, obtenu après 1 h d'incubation à 37°C est présenté dans la Figure 5B. Un marquage membranaire mais pas intracytoplasmique est observé lorsque les cellules sont incubées à 4°C avec rP40 (cf. Fig. 5A), alors qu'aucun marquage n'est observé en présence de glycophorine-Alexa (après incubation à 4°C comme à 37°C). L'exemple d'Alexa, molécule chimique, démontre que toute molécule chimique couplée à P40 peut ainsi être délivrée aux cellules présentatrices d'antigènes y compris les cellules dendritiques.

## Revendications

1. Utilisation d'une protéine OmpA d'entérobactérie ou d'un de ses fragments en association avec une substance biologiquement active dont l'efficacité est modulée et/ou liée à l'intemalisation de l'OmpA dans les cellules présentatrices d'antigènes pour la préparation d'une composition pharmaceutique destinée à prévenir ou à traiter les cancers, de préférence les cancers associés à un antigène tumoral, les maladies auto-immunes, les allergies, les rejets de greffes, les maladies cardiovasculaires, les maladies du système nerveux central, les maladies inflammatoires, les maladies infectieuses ou les maladies liées à une immunodéficience.

2. Utilisation selon la revendication 1, **caractérisée en ce que** ladite protéine OmpA d'entérobactérie ou l'un de ses fragments se fixe spécifiquement sur les cellules présentatrices d'antigènes.

3. Utilisation selon l'une des revendications 1 et 2, **caractérisée en ce que** lesdites cellules présentatrices d'antigènes sont choisies parmi les cellules dendritiques, les monocytes ou les lymphocytes B.

4. Utilisation selon la revendication 3, **caractérisée en ce que** lesdites cellules présentatrices d'antigènes sont les cellules dendritiques.

5. Utilisation selon l'une des revendications 1 à 4, **caractérisée en ce que** ladite protéine OmpA d'entérobactérie, ou l'un de ses fragments, est obtenue à partir d'une culture de ladite entérobactérie par un procédé d'extraction.

6. Utilisation selon l'une des revendications 1 à 4, **caractérisée en ce que** ladite protéine OmpA d'entérobactérie, ou l'un de ses fragments, est obtenue par voie recombinante.

7. Utilisation selon l'une des revendications 1 à 6, **caractérisée en ce que** ladite entérobactérie est Klebsiella *pneumoniae*.

8. Utilisation selon la revendication 7, **caractérisée en ce que** la séquence d'acides aminés de ladite protéine OmpA, ou l'un de ses fragments, comprend :
a) la séquence d'acides aminés de séquence SEQ ID N° 2 ;
b) la séquence d'acides aminés d'une séquence présentant un degré d'identité après alignement optimal d'au moins 80 % avec la séquence SEQ ID N° 2 ; ou
c) la séquence d'acides aminés d'un fragment d'au moins 5 acides aminés de la séquence SEQ ID N° 2 capable de se fixer spécifiquement sur les cellules présentatrices d'antigènes, lesdits fragments étant en outre capables d'être internalisés dans lesdites cellules présentatrices d'antigènes.

9. Utilisation selon l'une des revendications 1 à 8, **caractérisée en ce que** ladite substance biologiquement active est choisie parmi les peptides, les lipopeptides, les polysaccharides, les oligosaccharides, les acides nucléiques, les lipides et les substances chimiques.

10. Utilisation selon la revendication 9, **caractérisée en ce que** ladite substance biologiquement active est couplée par liaison covalente avec ladite protéine OmpA ou l'un de ses fragments.

11. Utilisation selon la revendication 10, **caractérisée en ce que** le couplage par liaison covalente est un couplage chimique.

12. Utilisation selon la revendication 11, **caractérisée en ce qu'**il est introduit un ou plusieurs éléments de liaison dans ladite protéine OmpA, ou l'un de ses fragments, et/ou de ladite substance biologiquement active pour faciliter le couplage chimique.

13. Utilisation selon la revendication 12, **caractérisée en ce que** ledit élément de liaison introduit est un acide aminé.

14. Utilisation selon la revendication 10, **caractérisée en ce que** ladite substance biologiquement active couplée par liaison covalente avec ladite protéine OmpA, ou l'un de ses fragments, est une protéine chimérique recombinante résultante de l'expression d'une construction d'acide nucléique codant pour ladite substance biologiquement active et pour ladite protéine OmpA, ou l'un de ses fragments.

15. Utilisation selon l'une des revendications 10 à 14, **caractérisée en ce que** ladite substance biologiquement active est un antigène ou un haptène.

16. Utilisation selon l'une des revendications 1 à 15, pour moduler la réponse immune vis-à-vis d'un antigène ou d'un haptène.

17. Utilisation selon la revendication 16, pour améliorer la réponse immune vis-à-vis d'un antigène ou d'un haptène.

18. Utilisation selon une des revendication 1 à 17, pour la préparation d'une composition pharmaceutique vaccinale destinée à prévenir ou à traiter une maladie infectieuse ou un cancer associé à un antigène tumoral.

19. Utilisation selon la revendication 18, **caractérisée en ce que** ladite composition pharmaceutique comprend en outre un adjuvant de l'immunité.

20. Utilisation selon l'une des revendications 18 et 19, **caractérisée en ce que** ladite composition pharmaceutique est véhiculée sous une forme permettant d'améliorer sa stabilité et/ou son immunogénicité.

21. Utilisation selon la revendication 20, **caractérisée en ce que** ladite composition pharmaceutique est véhiculée sous la forme d'un liposome.

22. Utilisation d'une cellule hôte transformée codant pour une protéine chimérique recombinante résultante de l'expression d'une construction d'acide nucléique codant pour une protéine OmpA d'entérobactérie ou d'un de ses fragments, et une substance biologiquement active consistant en un peptide et dont l'efficacité est modulée et/ou liée à l'internalisation de l'OmpA dans les cellules présentatrices d'antigènes pour la préparation d'une composition pharmaceutique destinée à prévenir ou à traiter les cancers, de préférence les cancers associés à un antigène tumoral, les maladies auto-immunes, les allergies, les rejets de greffes, les maladies cardiovasculaires, les maladies du système nerveux central, les maladies inflammatoires, les maladies infectieuses ou les maladies liées à une immunodéficience.

## Patentansprüche

1. Verwendung eines OmpA-Proteins eines Enterobacteriums oder von einem seiner Fragmente in Kombination mit einer biologisch wirksamen Substanz, deren Wirksamkeit durch die Internalisierung von OmpA in Antigen-präsentierende Zellen moduliert wird und/oder mit der Internalisierung von OmpA in Antigen-präsentierende Zellen verbunden ist, für die Herstellung einer pharmazeutischen Zusammensetzung, die dazu bestimmt ist, um Krebserkrankungen, vorzugsweise Krebserkrankungen, die mit einem Tumorantigen assoziiert sind, Autoimmunerkrankungen, Allergien, Transplantatabstoßungen, Herz-Kreislauf-Erkrankungen, Erkrankungen des Zentralnervensystems, entzündliche Erkrankungen, Infektionskrankheiten oder Krankheiten, die mit einer Immundefizienz verbunden sind, zu verhüten oder zu behandeln.

2. Verwendung nach Anspruch 1, **dadurch gekennzeichnet, dass** das OmpA-Protein eines Enterobacteriums oder das eine seiner Fragmente sich spezifisch an die Antigen-präsentierenden Zellen anheftet.

3. Verwendung nach einem der Ansprüche 1 und 2, **dadurch gekennzeichnet, dass** die Antigen-präsentierenden Zellen unter den dendritischen Zellen, den Monozyten oder den B-Lymphozyten ausgewählt werden.

4. Verwendung nach Anspruch 3, **dadurch gekennzeichnet, dass** die Antigen-präsentierenden Zellen dendritische Zellen sind.

5. Verwendung nach einem der Ansprüche 1 bis 4, **dadurch gekennzeichnet, dass** das OmpA-Protein eines Enterobacteriums oder das eine seiner Fragmente ausgehend von einer Kultur des genannten Enterobacteriums durch ein Extraktionsverfahren gewonnen wird.

6. Verwendung nach einem der Ansprüche 1 bis 4, **dadurch gekennzeichnet, dass** das OmpA-Protein eines Enterobacteriums oder das eine seiner Fragmente auf rekombinantem Wege erhalten wird.

7. Verwendung nach einem der Ansprüche 1 bis 6, **dadurch gekennzeichnet, dass** das Enterobacterium Klebsiella *pneumoniae* ist.

8. Verwendung nach Anspruch 7, **dadurch gekennzeichnet, dass** die Aminosäuresequenz des OmpA-Proteins oder des einen seiner Fragmente umfasst:
a) die Aminosäuresequenz der Sequenz SEQ ID NO. 2;
b) die Aminosäuresequenz einer Sequenz, die nach optimaler Ausrichtung ("alignment") ein Identitätsausmaß von wenigstens 80% zu der Sequenz SEQ ID NO. 2 aufweist;
c) die Aminosäuresequenz eines Fragments von wenigstens 5 Aminosäuren der Sequenz SEQ ID NO. 2, das in der Lage ist, sich spezifisch an Antigen-präsentierende Zellen anzuheften, wobei die Fragmente außerdem in der Lage sind, in die genannten Antigen-präsentierenden Zellen internalisiert zu werden.

9. Verwendung nach einem der Ansprüche 1 bis 8, **dadurch gekennzeichnet, dass** die biologisch wirksame Substanz unter den Peptiden, Lipopeptiden, Polysacchariden, Oligosacchariden, Nukleinsäuren, Lipiden und den chemischen Substanzen ausgewählt wird.

10. Verwendung nach Anspruch 9, **dadurch gekennzeichnet, dass** die biologisch wirksame Substanz mit dem Protein OmpA oder dem einen seiner Fragmente durch kovalente Bindung gekoppelt ist.

11. Verwendung nach Anspruch 10, **dadurch gekennzeichnet, dass** die Kopplung durch kovalente Bindung eine chemische Kopplung ist.

12. Verwendung nach Anspruch 11, **dadurch gekennzeichnet, dass** in das OmpA-Protein oder das eine seiner Fragmente und/oder die biologisch wirksame Substanz ein oder mehrere Bindungselemente eingeführt worden sind, um die chemische Kopplung zu erleichtern.

13. Verwendung nach Anspruch 12, **dadurch gekennzeichnet, dass** das eingeführte Bindungselement eine Aminosäure ist.

14. Verwendung nach Anspruch 10, **dadurch gekennzeichnet, dass** die biologisch wirksame Substanz, die durch kovalente Bindung mit dem OmpA-Protein oder dem einen seiner Fragmente gekoppelt ist, ein chimäres rekombinantes Protein ist, das aus der Expression eines Nukleinsäurekonstrukts, das die biologisch wirksame Substanz und das OmpA-Protein oder das eine seiner Fragmente kodiert, resultiert.

15. Verwendung nach einem der Ansprüche 10 bis 14, **dadurch gekennzeichnet, dass** die biologisch wirksame Substanz ein Antigen oder ein Hapten ist.

16. Verwendung nach einem der Ansprüche 1 bis 15, um die Immunantwort gegenüber einem Antigen oder einem Hapten zu modulieren.

17. Verwendung nach Anspruch 16, um die Immunantwort gegenüber einem Antigen oder einem Hapten zu verbessern.

18. Verwendung nach einem der Ansprüche 1 bis 17 für die Herstellung einer pharmazeutischen Impf-Zusammensetzung, die dazu bestimmt ist, eine Infektionskrankheit oder eine mit einem Tümorantigen assoziierte Krebserkrankung zu verhüten oder zu behandeln.

19. Verwendung nach Anspruch 18, **dadurch gekennzeichnet, dass** die pharmazeutische Zusammensetzung außerdem ein Immunitäts-Adjuvans umfasst.

20. Verwendung nach einem der Ansprüche 18 und 19, **dadurch gekennzeichnet, dass** die pharmazeutische Zusammensetzung mit einem Träger kombiniert ist in einer Form, die es erlaubt, deren Stabilität und/oder deren Immunogenität zu verbessern.

21. Verwendung nach Anspruch 20, **dadurch gekennzeichnet, dass** die pharmazeutische Zusammensetzung mit einem Träger kombiniert ist in Form eines Liposoms.

22. Verwendung einer transformierten Wirtszelle, die ein chimäres rekombinantes Protein kodiert, das aus der Expression eines Nukleinsäurekonstrukts resultiert, das ein OmpA-Protein eines Enterobacteriums oder eines seiner Fragmente und eine biologisch wirksame Substanz, die aus einem Peptid besteht und deren Wirksamkeit durch die Internalisierung von OmpA in Antigen-präsentierende Zelle moduliert wird und/oder mit der Internalisierung von OmpA in Antigen-präsentierende Zellen verbunden ist, kodiert, für die Herstellung einer pharmazeutischen Zusammensetzung, die dazu bestimmt ist, Krebserkrankungen, vorzugsweise Krebserkrankungen, die mit einem Tumorantigen assoziiert sind, Autoimmunerkrankungen, Allergien, Transplantatabstoßungen, Herz-Kreislauf-Erkrankungen, Erkrankungen des Zentralnervensystems, entzündliche Erkrankungen, Infektionskrarikheiten oder Krankheiten, die mit einer Immundefizienz verbunden sind, zu verhüten oder zu behandeln.

## Claims

1. Use of an enterobacterium OmpA protein, or of a fragment thereof, in combination with a biologically active substance the effectiveness of which is modified by and/or linked to the internalization of OmpA into antigen-presenting cells, for preparing a pharmaceutical composition intended to prevent or to treat cancers, preferably cancers associated with a tumor antigen, autoimmune diseases, allergies, graft rejections, cardiovascular diseases, diseases of the central nervous system, inflammatory diseases, infectious diseases or diseases linked to an immunodeficiency.

2. Use according to Claim 1, **characterized in that** the said enterobacterium OmpA protein, or a fragment thereof, binds specifically to antigen-presenting cells.

3. Use according to either of Claims 1 and 2, **characterized in that** the said antigen-presenting cells are chosen from dendritic cells, monocytes and B lymphocytes.

4. Use according to Claim 3, **characterized in that** the said antigen-presenting cells are dendritic cells.

5. Use according toone of Claims 1 to 4, **characterized in that** the said enterobacterium OmpA protein, or a fragment thereof, is obtained from a culture of the said enterobacterium, using an extraction process.

6. Use according to one of Claims 1 to 4, **characterized in that** the said enterobacterium OmpA protein, or a fragment thereof, is obtained by recombinant process.

7. Use according to one of Claims 1 to 6, **characterized in that** the said enterobacterium is Klebsiella *pneumoniae*.

8. Use according to Claim 7, **characterized in that** the amino acid sequence of the said OmpA protein, or a fragment thereof, comprises:
a) the amino acid sequence of sequence SEQ ID No 2;
b) the amino acid sequence of a sequence having a degree of identity, after optimal alignment, of at least 80% with the sequence SEQ ID No 2; or
c) the amino acid sequence of a fragment, of at least 5 amino acids, of the sequence SEQ ID No. 2 capable of binding specifically to antigen-presenting cells, the said fragments also being capable of being internalized into the said antigen-presenting cells.

9. Use according to one of Claims 1 to 8, **characterized in that** the said biologically active substance is chosen from peptides, lipopeptides, polysaccharides, oligosaccharides, nucleic acids, lipids and chemical substances.

10. Use according to Claim 9, **characterized in that** the said biologically active substance is coupled by covalent attachment with the said OmpA protein, or a fragment thereof.

11. Use according to Claim 20, **characterized in that** the coupling by covalent attachment is chemical coupling.

12. Use according to Claim 11, **characterized in that** one or more attachment elements is (are) introduced into the said OmpA protein, or a fragment thereof, and/or into the said biologically active substance, in order to facilitate the chemical coupling.

13. Use according to Claim 12, **characterized in that** the said attachment element introduced is an amino acid.

14. Use according to Claim 10, **characterized in that** the said biologically active substance coupled by covalent attachment with the said OmpA protein, or a fragment thereof, is a recombinant chimeric protein resulting from the expression of a nucleic acid construct encoding the said biologically active substance and the said OmpA protein, or a fragment thereof.

15. Use according to one of Claims 10 to 14, **characterized in that** the said biologically active substance is an antigen or a hapten.

16. Use according to one of Claims 1 to 15, for modifying the immune response against an antigen or a hapten.

17. Use according to Claim 16, for improving the immune response against an antigen or a hapten.

18. Use according to one of Claims 1 to 17, for preparing a pharmaceutical vaccine composition intended to prevent or to treat an infectious disease or a cancer associated with a tumor antigen.

19. Use according to Claim 18, **characterized in that** the said pharmaceutical composition also comprises an adjuvant of immunity.

20. Use according to either of Claims 18 and 19, **characterized in that** the said pharmaceutical composition is vehicled in a form which makes it possible to improve the stability and/or immunogenicity thereof.

21. Use according to Claim 20, **characterized in that** the said pharmaceutical composition is vehicled in the form of a liposome.

22. Use of a transformed host cell encoding a recombinant chimeric protein resulting from the expression of a nucleic acid construct encoding an enterobacterium OmpA protein, or a fragment thereof, and a biologically active substance consisting of a peptide and the effectiveness of which is modified by and/or linked to the internalization of OmpA into antigen-presenting cells, for preparing a pharmaceutical composition intended to prevent or to treat cancers, preferably cancers associated with a tumor antigen, autoimmune diseases, allergies, graft rejections, cardiovascular diseases, diseases of the central nervous system, inflammatory diseases, infectious diseases or diseases linked to an immunodeficiency.
